# EUROPEAN PATENT APPLICATION

(11) **EP 0 995 483 A1**
(43) Date of publication of application: **26.04.2000**
(21) Application number: 98203574.3
(22) Date of filing: 23.10.1998
(51) Int. Cl.: B01D 61/14, C02F 1/44, B01D 46/00, D06F 39/10, A47L 15/42, B01J 19/24, C12M 1/12, C25D 13/24, A61M 1/34

(54) **A cross-flow filtration apparatus**

(71) Applicant: THE PROCTER & GAMBLE COMPANY, Cincinnati, Ohio 45202 (US)
(72) Inventor: The designation of the inventor has not yet been filed
(74) Representative: O'Connell, Maura

(57) **Abstract**

The present invention concerns a cross-flow filtration apparatus (10) comprising a filtration means (50) having a feed inlet means (18,22), a permeate outlet means (16) and a retentate outlet means (20).

The feed inlet means of the apparatus (10) includes a first conduit (18) in fluid communication from a process zone (12) to a buffer zone (14) and a second conduit (22) in fluid communication from the buffer zone (14) to the filtration means (50).

The retentate outlet means (20) is in fluid communication with the buffer zone (14) and the permeate outlet means (16) is in fluid communication with the process zone (12).

In use, substantially undiluted permeate is accumulated in the process zone (12), whilst a suspension of retained matter is accumulated in the buffer zone (14).

The apparatus (10) of the present invention is suitable for treating wash liquor from laundry or dishwashing machines or the like.

## Description

The present invention is based on cross-flow filtration. In particular, the present invention concerns a cross-flow filtration apparatus, which allows continuous *in-situ* filter action of process fluid as well as automated and separate harvesting of substantially undiluted permeate and, if desired, of retentate. The permeate is re-directed to the original process zone, making it possible to achieve virtually no process volume dilution. Serial interconnection of several such cross-flow filtration apparatuses allows process liquid purification by different parameters; thereby facilitating particulates removal or macromolecule removal as desired.

According to a first aspect of the present invention, there is provided a cross-flow filtration apparatus (10) suitable for connection to a process zone (12) for filtration of process fluid, the apparatus comprising a cross-flow filtration means (50) having a feed inlet means (18, 22), a permeate outlet means (16) and a retentate outlet means (20) in fluid communication therewith, characterised in that the apparatus (10) comprises a buffer zone (14); in that the feed inlet means (18, 22) includes first and second conduits, the first conduit (18) being in fluid communication from the process zone (12) to the buffer zone (14), and the second conduit (22) being in fluid communication from the buffer zone (14) to the filtration means (50); in that the retentate outlet means (20) is in fluid communication with the buffer zone (14); and in that the permeate outlet means (16) is in fluid communication with the process zone (12).

The process zone (12) can generally take the form of a reaction zone, a cleaning zone, a coating zone, a living zone for humans or other animals, a treatment zone, a manufacturing zone, a storage zone and/or a feed zone. In preferred embodiments, the process zone (12) is a reservoir (sometimes referred to herein as "the second reservoir") which can be open or closed to atmosphere, although open reservoirs are preferred herein. Process zones other than reservoirs are also envisaged, such as, for example, tubular reaction zones, surface treatment zones, etc. Importantly, the process zone (12) acts both as feed source and as output sink for the cross-flow filtration means (50).

The primary function of the buffer zone (14), on the other hand, is to act as a storage and/or accumulation zone for the retentate and, as such, the buffer zone (14) will normally take the form of a reservoir (sometimes referred to herein as "the first reservoir"). It will be understood, however, that the buffer zone (14) can also act as a reaction, cleaning, treatment zone, etc., for the process fluid and/or retentate and is thus not limited to a storage functionality.

Preferably, the buffer zone (14) is adapted and arranged such that, at steady-state, outflow along the permeate outlet means (16) is substantially equivalent (mass-balanced) to inflow along the first conduit (18).

More preferably, the buffer zone (14) is substantially closed to atmosphere.

Even more preferably, the retentate outlet means (20), the buffer zone (14) and the second conduit (22) form, with the filtration means (50), a normally closed, but bleedable, retentate recirculating loop (70).

Advantageously, the filtration means (50) is adapted for filtration of liquids and is, preferably, selected from a microfiltration, ultrafiltration or reverse osmosis filtration means (50).

Alternatively, the filtration means (50) is adapted for filtration of gases.

More advantageously, two or more first conduits (18), buffer zones (14), second conduits (22), filtration means (50), retentate outlet means (20) and permeate outlet means (16) are in fluid communication with one process zone (12).

Even more advantageously, a valve (40) is provided in operative association with the two or more first conduits (18).

Preferably, the process zone (12) comprises liquid conduits of a laundry or dishwashing machine or an aquarium.

According to a second aspect of the invention, there is provided a domestic appliance, preferably a laundry or dishwashing machine incorporating a cross-flow filtration apparatus (10) according to a first aspect of the present invention.

Other applications of the cross-flow filtration equipment herein include chemical reactors, enzyme reactors, bioreactors, electrocoating and electroplating equipment, blood/plasma fractionation/filtration equipment and air/water/effluent treatment equipment.

It will be understood that cross-flow filtration apparatus comprising only a single reservoir (buffer zone) is also envisaged herein, the apparatus being connected to an external process zone at the point of use.

Thus, according to a further aspect of the invention, there is provided use of a cross-flow filtration apparatus (10) suitable for connection to a process zone (12) for the filtration of process fluid, the apparatus comprising a buffer zone (14), a cross-flow filtration means (50) having a feed inlet means (18, 22), a permeate outlet means (16) and a retentate outlet means (20) in fluid communication therewith, and wherein the feed inlet means (18, 22) includes first and second conduits, characterised in that in use, the first conduit (18) is in fluid communication from the process zone (12) to the buffer zone (14), the second conduit (22) is in fluid communication from the buffer zone (14) to the filtration means (50), the retentate outlet means (20) is in fluid communication with the buffer zone (14), and the permeate outlet means (16) is in fluid communication with the process zone (12).

The term "cross-flow filtration" requires that flow through the filter medium (permeate) be perpendicular to the feed flow, the flow alongside the filter medium from the filtration means being defined as retentate. An additional feature of cross-flow filtration is that particle polarisation is prevented by the shear induced by the flow rate. In contrast, through-flow filtration (sometimes called dead-ended filtration) concerns filtration in which the permeate and feed flow directions are the same. Through-flow filtration requires frequent back-washing, in order to allow filtration to continue.

Reference is made to "Handbook of Industrial Membrane Technology" (ed. Mark C. Porter; Noyes Publications, New Jersey, U.S.A., 1990), the whole contents of which are incorporated herein, for a complete discussion of cross-flow filtration and its applications.

The present invention concerns the general field of cross-flow filtration for separations involving domestic, industrial or municipal process streams. Most of such membrane separation processes are pressure driven, the main exception being electrodialysis, whereby ions are separated under the influence of an electric field.

The pressure driven liquid cross-flow filtration processes, specifically microfiltration, ultrafiltration and reverse osmosis, may be distinguished by the size of particle/molecule the membrane is capable of retaining or passing. This, in turn, roughly relates to the pore-size of the membrane. An arbitrary dividing line between microfiltration and ultrafiltration would be at 1,000Å. Thus, microfiltration is capable of retaining pollen, starch, blood cells and bacteria. Again, the dividing line between ultrafiltration and reverse osmosis is somewhat semantic at about 10Å. Thus, ultrafiltration is capable of retaining DNA, viruses, albumin, Vitamin B₁₂ and glucose, whilst reverse osmosis is capable of retaining water and salts.

The present invention has particular utility in continuous filter action processes which allow harvesting of both substantially undiluted permeate and retentate without the need to interrupt the manufacturing, cleaning or treatment process. Thus, for a microfiltration apparatus of the present invention, materials such as pollen, starch, blood cells, bacteria, heavy metals and the like may be continuously harvested from a manufacturing or process medium. For an ultrafiltration apparatus of the present invention, materials such as DNA, viruses, albumin, glucose and the like may be continuously harvested, whilst a reverse osmosis apparatus of the present invention may be used to continuously harvest water, salts and the like.

Ultrafiltration and reverse osmosis usually use cross-flow filtration to sweep away deposited particles from the membrane surface, whilst most microfiltration systems use through-flow filtration, so that the particles accumulate on the membrane and are disposed of with the membrane. However, microfiltration systems can also operate using cross-flow filtration.

The pressure driven gas filtration processes include pervaporation and gas separations. Pervaporation is a special form of gas separation in that it is a concentration driven process - the feed mixture is supplied as a liquid to the membrane and the permeate is recovered as a vapour on the low pressure side of the membrane.

Microfiltration can be traced back to the 19th century with the development of the first nitrocellulose membranes in 1855. Even today, the most common polymers used in microfiltration membranes are mixed esters of cellulose. Eventually, membranes made of materials other than cellulose nitrate began to appear such as, for example, cellulose triacetate, regenerated cellulose, polyvinyl chloride, polyamide, polycarbonate, silver membrane, polypropylene, polytetrafluoroethylene, polysulfone, polyvinylidene fluoride, polyester and alumina.

All current microfiltration membranes may be classified as either "tortuous-pore" or "capillary-pore" membranes, both of which may be used in the apparatus of the present invention. The "tortuous-pore" structure resembles a sponge with a network of interconnecting tortuous pores, whilst the "capillary-pore" structure is distinguished by its straight-through cylindrical capillaries. The "tortuous-pore" membranes are the more common and include typical cellulosic membranes and virtually all other polymers. The "capillary-pore" membranes are available as polycarbonate or polyester membranes supplied by Nuclepore Corporation and by Poretics Corporation.

Tortuous-pore membranes are usually made by a process known as phase inversion. Alternatively, "tortuous-pore" membranes may be made by a stretching process or by a thermal-phase-inversion process.

Capillary-pore membranes may be made using laser beams, electroforming, photochemical etching and ionotropy, so as to orientate anisotropic gel particles to form ionotropic-gel membranes. The "track etch" process, described in US-A-3, 303, 085, produces submicron capillary-pore membranes.

Internal pore fouling is negligible when a "capillary-pore" membrane is used to filter particles which are considerably larger then the membrane pore size - the sweeping action of the cross-flow stream tangential to the membrane surface maintains a stable flux at constant concentration. However, for particles with sizes somewhat nearer the membrane pore size, some internal pore fouling will occur, but at a greatly reduced rate. Using cross-flow filtration with a "tortuous-pore" membrane, the permeate flux declines rapidly at first, as boundary layer conditions are established and then levels off with a diminishing rate of permeate flux decay.

Permeate flux decay due to internal pore fouling can often be relieved by back flushing or chemical cleaning but the ultimate solution is to develop microfiltration membranes with a high degree of anisotropy, so that the membrane surface facing the feed stream has exceedingly small pores preventing blockage, thereby, of the internal pores.

Microfiltration membranes usually take the form of plate and frame units; pleated cartridges; or tubular/hollow-fibre modules, the use of all of which falls within the scope of the invention.

Plate and frame units suitable for cross-flow filtration can take sheet stock microfiltration membranes. Alternatively, the plate and frame unit may take a pre-assembled membrane cassette, which is a sandwich of two outer layers of membranes sealed to an inner filtrate collection screen. In that event, the plate and frame arrangement is formed by placing a cross-flow spacer between the adjacent filter packets which are then stacked in the plate and frame arrangement.

Pleated cartridges are also suitable for use in cross-flow microfiltration. In the simplest embodiment, a sleeve may be placed around the cartridge and the housing modified, so as to withdraw the retentate stream from the bottom of the housing, the cross-flow stream being thereby forced into the pleats where it moves tangential to the membrane.

Tubular/hollow-fibre modules comprise "tortuous-pore" membranes. Suitable tubular modules include membranes composed of alumina, polypropylene or polyvinylidene difluoride and suitable hollow-fibre modules are composed of cellulose ester, polypropylene, polysulfone or polyvinyl alcohol.

Many of the applications for microfiltration derive from their excellent retention of microorganisms. Examples of cross-flow filtration applications include removal of heavy metals from waste waters; plasmapheresis; cell harvesting/washing; continuous cell culture; and prefiltration for ultrafiltration.

The commercial beginnings of ultrafiltration date back to the early 1960s, when the techniques used to make asymmetric reverse osmosis membranes were discovered to also be applicable to the production of high-flux ultrafiltration membranes.

A typical ultrafiltration membrane has a thin skin on its surface, which permits high hydraulic permeability with a more open porous substructure which provides good mechanical support. Additional strength may be provided by casting the membrane on a spun-bonded polyethylene or polypropylene backing.

Cellulose acetate and polyelectrolytes were among the first synthetic polymers to be used for ultrafiltration membranes. Today, ultrafiltration membranes may be made from a variety of synthetic polymers, including polyvinyl chloride, polyacrylonitrile, polycarbonate, aliphatic and aromatic polyamides, polyimides, polysulfone, polyarylsulfone and polyvinylidene difluoride, as well as inorganic sources such as zirconium and aluminium oxides.

Suitable ultrafiltration membranes may be cast as flat-sheets, as tubes or as hollow fibres, the use of all of which are within the scope of the present invention.

Permeate flux may be augmented either by centrifugal forces for particles having a density higher than water or by electric fields for particles bearing an electric charge. Thus, where the centrifugal force vector is perpendicular to the membrane surface but opposite (and parallel) to the flux vector, significant permeate flux improvements can be observed. Similarly, for particles bearing a charge, the application of an electric field can cause such species to migrate away from the membrane, thereby augmenting the mass transfer co-efficient and augmenting the permeate flux observed.

In cross-flow electrofiltration, it is desired that the field strength be below the voltage at which net particle migration toward the ultrafiltration membrane is zero and, in this event, increases in tangential velocity result in a higher permeate flux.

The ultrafiltration membrane configuration is application-dependent and generally includes four generic configurations, namely, tubes; hollow fibres; plate and frame units; and spiral wound modules, the use of all of which is within the scope of the present invention.

Tubes are, perhaps, the simplest configuration, in which the membrane is cast on the inside wall of a porous support tube. However, the biggest disadvantage of tubes is their cost, the porous support tube itself being the dominant cost factor.

Hollow fibres are, in theory, the ideal membrane configuration in that there is no "parasite" drag and no expensive porous support tube. Such fibres can be pressurised on the inside permitting "thin channel" fluid management of the feed stream. However, the biggest disadvantage of hollow fibres is the pressure constraint which limits the cross-flow velocity down the lumen of the fibre. In addition, the hollow fibre configuration is more susceptible to fouling and plugging than the other three configurations and larger diameter fibres are becoming popular to improve fouling resistance. Fortunately, hollow fibres can be readily cleaned by back-washing, which tends to compensate for their propensity to foul. In contrast, it is not recommended that tubes; plate and frame units; and spiral wound modules be back-washed, due to problems with membrane delamination and glue line seal rupture. Flat sheet membranes in a plate and frame unit offer the greatest versatility but at the highest financial cost.

Spiral wound modules were originally developed for reverse osmosis, but are capturing an increasing share of the ultrafiltration market by providing one of the least expensive ultrafiltration modules available in terms of cost per membrane area unit. Spiral wound units cannot be unwrapped for cleaning and most cannot be autoclaved. In terms of propensity to fouling, they are intermediate between hollow fibres on the one hand and, on the other hand, tubes and some plate and frame units.

The production of ultrapure water (as permeate) has applications, for example, in the semiconductor industry and in the pharmaceutical industry. Thus, yields of integrated circuits are improved, when the water quality is improved. Similarly, it is crucial that all microorganisms, pyrogens and other particles be removed from water for pharmaceutical injection purposes.

The electrocoat paint market forms the largest single application for ultrafiltration. Car plants usually use electrocoat as the undercoat. Ultrafiltration may be employed to reduce pollution problems and to reduce paint loss, by recycling electrocoat-containing permeate and by discarding retentate. However, it is not known to recycle such electrocoat-containing permeate to a process zone, whilst simultaneously transferring retentate to a buffer zone, as is called for in the invention in suit.

Billions of litres of oily waste water are generated every day. Strict environmental legislation now requires industries to clean up such waste water. Industrial oily waste water can be divided into three broad categories, namely, free-floating oil, unstable oil-water emulsions and stable oil-water emulsions, of which stable oil-water emulsions are most difficult to deal with and, at the same time, the most amenable to ultrafiltration. Stable oil - water emulsions are generated in such diverse industries as metal working, metal cleaning, rolling and drawing, food processing and textile manufacturing.

Waste lubricating oil can be refined into clean lubricants (as permeate) by ultrafiltration which can remove all contaminants (as retentate), although the flux is very low at room temperature.

Crude oil contains metals, either chemically bound or associated with asphaltenes, and ultrafiltration can retain such metal asphaltene complexes whilst passing virtually all other crude oil factions as permeate. Again, the flux is low due to the high viscosity.

Warp sizing agents are used to improve the strength and surface characteristics of warp ions prior to weaving operations and recovery of such synthetic sizing agents as polyvinyl alcohol and sodium carboxymethylcellulose can be achieved by ultrafiltration. Similarly, low molecular weight dyes such as indigo can be recovered by way of ultrafiltration or reverse osmosis. Ultrafiltration can also remove heavy metals from metal plating wastes and lignin compounds and colour bodies from waste in the pulp and paper industry. Ultrafiltration can also be used to recover cheese whey protein, soy whey, egg white or gelatin; to increase fruit juice recovery; to sterilise wine; and to improve beer clarity.

There is a wide diversity of applications for ultrafiltration in the pharmaceutical and biotechnological industries. Most industrial biological syntheses are carried out on a batch scale with a small amount of product recovered from large quantities of solvent and ultrafiltration is most useful in the recovery and/or concentration and purification of such products. Thus, ultrafiltration may be used to harvest cells, as in the case of microfiltration described hereinabove; concentrate and purify enzymes, by removal of small peptides, etc.; process blood plasma to obtain purified therapeutic proteins; diafiltration (removal of salts and/or lower molecular weight species from larger macromolecules); or concentrate viruses and hormones and materials of similar size.

In addition, ultrafiltration membranes can be used in the processing of pharmaceuticals and biologicals by, for example, using enzyme reactors or membrane fermentors, the use of both of which fall within the scope of the present invention.

In an enzyme reactor, the enzyme is immobilised on a membrane. Three types of enzyme reactors, for ultrafiltration membranes, can be mentioned, namely, mobile enzyme, immobilised enzyme and disperse solubilised immobilised enzyme, the use of all of which falls within the scope of the present invention. In the mobile enzyme version, the enzyme is retained by an ultrafiltration membrane which is permeable to the products of the reaction. Thus, retentate and enzyme are recirculated, but enzyme may become inactivated due to the continuous pumping and recirculation through the ultrafiltration unit. In the immobilised version, the enzyme is immobilised within the ultrafiltration membrane. The immobilised version can be used in a back flush mode or in a recycle mode. In the disperse soluble immobilised enzyme version, enzymes are immobilised onto mobile supports, whereupon improved stability, but decreased access of the enzyme to the substrate, is observed.

A membrane fermentor is an enzyme reactor using intracellular, rather than extracellular, enzymes. There are two main types of membrane fermentors, namely, mobile cells and immobilised cells, the use of both of which falls within the scope of the present invention. In the mobile cell embodiment, the ultrafiltration apparatus retains the biomass, whilst the products of the fermentation and, in addition, metabolic waste products are continuously withdrawn through the membrane. Alternatively, the cells can be immobilised in the walls of the ultrafiltration hollow fibres, so that products such as interferon, monoclonal antibodies, antigens, viruses and hormones may be produced continuously.

Industrial applications of reverse osmosis date back to 1970 or so. Now, most electronics plants would use reverse osmosis as pre-treatment in the preparation of ultrapure water.

Reverse osmosis finds applications predominantly in industrial areas where it is used to prepare industrial process water or to process wastes; irrigation where it is used to upgrade waters for agricultural purposes; municipal, where it is used to upgrade waters to municipal drinking water levels; and power, where it is used to prepare process water in electric power stations.

Typical membrane configurations for reverse osmosis include plate and frame; spiral wound; tubular; and hollow, fine fibre, the use of all of which are contemplated in the present invention.

The plate and frame configuration is much like a conventional plate and frame configuration, except that, in reverse osmosis, a higher fluid operating pressure is typically used. Such configurations have the advantage that only the membrane itself must be replaced when a membrane becomes defective but suffer from the disadvantage of complex flow patterns and high costs.

The spiral wound membrane configuration has the advantage of high packing density and high flux, which makes it one of the more cost effective elements, but the disadvantage that a moderate amount of pre-treatment is required for some feed waters to prevent fouling of the spacers.

The tubular reverse osmosis device has the advantage of being able to tolerate high suspended solids concentrations in the feed and the possibility of mechanical membrane cleaning, but the disadvantages are the excessive number of tube end fittings in proportion to the active membrane area in each pressure vehicle; the bulkiness of the overall configuration; and the high cost.

Hollow, fine, fibrous, synthetic filaments have the advantage of high packing density and the elimination of the membrane support materials, but the main disadvantage is the need for an efficient feed water pre-treatment, to remove suspended and colloidal solids.

Reverse osmosis membranes can also take the form of dynamic membranes which are formed by feeding a feed solution containing the membrane-forming material tangential to a clean porous surface, thereby quickly forming the dynamic membrane. Such dynamic membranes may have applications for, for example, orange juice concentration and promise high flux with lower rejection and a low membrane cost.

Known existing cross-flow filtration apparatus system designs are:

### Single Pass Filtration

Process liquid is fed, using a pump, via the feed inlet means, to the cross-flow filtration apparatus and permeate is collected. Unlike the batch process, the retentate is not returned to the process liquid reservoir. Only a small increase in concentration is achieved. Single-pass ultrafiltration can only be used with relatively pure feed streams such as, for example, deionised water where, if the retained matter is low in concentration, the permeate recovery can be as much as 95-99% of the feed.

However, in most cases, the flux is too low to operate in a single pass mode, so that recirculation of the process stream across the membrane is necessary to obtain the desired concentration or recovery. Such recirculation can be accomplished by either a batch concentration or by a feed and bleed operating mode.

### Batch Process

Process liquid is fed, using a pump, from a feed reservoir via the feed inlet means, to the cross-flow filtration apparatus. Retentate is recirculated back to the feed reservoir; permeate is fed away from the recirculation loop via a permeate outlet means and is collected. As permeate is removed via the permeate outlet means, the volume in the feed reservoir drops and the concentration of retained species rises, whilst freely permeable species, for example, salts, remain at the same concentration both in the feed reservoir and in the permeate outlet stream. Eventually, the volume in the feed reservoir becomes too small to pump and the batch run is over. A new batch must be charged to the feed reservoir, in order to continue. Thus, the net effect is a rapid concentration of retained solids in the process volume, whose volume decreases during the batch filtration process.

### Feed and Bleed Process

Process liquid, as in the batch process, is continuously fed into the system, the difference being that the feed reservoir is not in line in the recirculating loop, the feed stream being merely fed into the recirculating loop. Permeate is again collected and retentate is recirculated. The concentration of retained species continues to increase with an ever decreasing flux, unless a purge stream is taken from the recirculating loop. A ratio controller may be used to keep the feed to bleed ratio equal to the concentration ratio required so that, even with flux decay, the concentration ratio and recovery will remain constant. The disadvantage is that the separation of the bleed liquid is not complete.

### Diafiltration Process

As in the batch system, process liquid is fed from a process liquid reservoir to the cross-flow filtration apparatus, using a pump. Dilute permeate is fed out of the recirculation loop and is collected; retentate is recirculated to the process liquid reservoir, which is closed to atmosphere. Unlike the batch system, the permeate volume removed by the filtration means, is replaced by fresh solvent (feed solvent), which is fed into the process liquid reservoir from a feed solvent reservoir. Thus the initial process liquid volume is maintained and less filter clogging is observed. Complete separation is possible if enough diafiltration feed solvent is allowed to pass the system.

Of all of the above plant designs, the diafiltration approach is most effective to eliminate, for example, insoluble matter from detergent matrix solutions. However, the disadvantage is the large end volume of permeate. Typically a diafiltration feed volume to process volume ratio of 4:1 is required to obtain sufficient separation. This, in turn, means that a concentration step must be carried out on the dilute permeate before moving to the next purification process. Such a concentration step is tedious and time consuming.

Thus, conventional cross-flow diafiltration focuses on the elimination of permeate or non-retained matter from the system. The present invention revolves, instead, around inverting this, in that retentate or retained matter is concentrated in a buffer zone (first reservoir) and substantially undiluted permeate or non-retained matter is directed back to the process zone (original process fluid container or second reservoir).

The harvesting of substantially undiluted permeate enabled by the present invention opens up a wide variety of potential applications, including analytical purification of detergent matrices and isolation of e.g. polymeric compounds; in-line process fluid clean up (e.g. automatic laundry or dish washing machines); and continuous removal of by-products or end-products in laboratory-scale, pilot plant scale or industrial scale processes.

Polymers are important ingredients in current detergent formulations. Ultrafiltration and diafiltration are frequently employed for characterisation and identification of such polymers. For detergent matrices, a particular problem arises with conventional cross-flow filtration apparatus system designs - current detergent formulations may contain up to 30% insoluble materials, mostly, zeolites, which must be removed before attempting polymer isolation. Currently this is achieved by a dead-ended batch filtration on a flat glass fibre (1µm) filter. This is not satisfactory, as the filter materials clog and filtration may take several hours, requiring much manual manipulation.

In contrast, the present invention concerns a cross-flow filtration apparatus which permits automated quantitative solids separation (for example, zeolites) from a detergent formulation without accumulation of solids on the membrane surface and substantially quantitative recovery of substantially undiluted permeate.

Although the invention is described and exemplified in relation to filtration of liquid detergent formulations, it is by no means limited to particulate filtration, but covers the range of micro and ultra filtration as well as reverse osmosis. In addition, the invention covers the range of gas cross-flow filtration.

The benefits of the present cross-flow filtration apparatus are not limited to analytical applications, but can be expanded to e.g. pilot plant scale or process scale purification or it can be built, in-line, in industrial equipment or in home appliances such as laundry or dish washing machines.

An important advantage of the present invention is that, because retained matter is fed to a remote location separate from the process zone (second reservoir or process fluid tank), the process zone, the first conduit, the second conduit and/or the filtration means itself, can be fitted with optional devices for selective processing of the process fluid and/or retentate, e.g. to improve reaction kinetics. Suitable optional devices include, but are not limited to, a UV source outside the process zone or the modification of the filtration membrane with, for example, enzymes or other reactants as described hereinabove. Positioning of a UV source outside the process zone avoids unwanted UV irradiation of components inside the process zone and immobilising enzymes or other reactants outside the process zone permits added control over process reactions.

Alternatively, the basic system design can be used to remove or release reactants to the process zone in a controlled manner, by a time controlled operation of the unit.

The most important feature of the present cross-flow filtration apparatus is its "reversed" operation - whilst conventional systems eliminate permeate or non-retained matter from the system and concentrate retained matter in the process zone (process fluid tank), the present apparatus continuously re-directs permeate or non-retained matter back to the process zone (second reservoir or process fluid tank) whilst retained matter is kept in a slurry in the buffer zone (first reservoir). As a result, the filtration process of the present invention makes it possible to produce "clean" substantially undiluted process fluid (process fluid substantially free of retained matter) in the process zone (original process fluid tank or second reservoir) and a concentrated slurry of retained matter, remote from the process zone, in the buffer zone (first reservoir). This is in direct contrast to the conventional filtration system, where the filtration process has to be stopped when retained matter is concentrated in the process fluid tank to such an extent that the pump fails to recirculate the mixture, or the filtration membrane ruptures.

Results of initial tests have indicated that the present approach lends itself very well to the pre-purification of detergent solutions prior to more specific clean-up procedures.

The individual components of the cross-flow filtration apparatus of the present invention are commercially available. The invention revolves, in one aspect, around solving the dilution issue, created by constant addition of feed solvent which eventually ends up as permeate.

The invention will now be illustrated with reference to the accompanying drawings, in which:-
Figure 1 shows a schematic representation of a cross-flow filtration apparatus of the present invention;
Figure 2 shows a schematic representation of two cross-flow filtration apparatuses of the present invention;
Figure 3 shows a schematic representation of the cross-flow filtration apparatus of Figure 1, in which a second reservoir (process zone) is replaced by a washing machine;
Figure 4 shows filtration performance for the apparatus of Example 1;
Figures 5-6 show filtration performance for the apparatus of Example 2;
Figures 7-10 show filtration performance for the apparatus of Example 3;
Figure 11 shows the filtration performance for the apparatus of Example 4;
Figure 12 shows a cross-flow filtration apparatus of the present invention of Example 5; and
Figure 13 shows filtration performance for the apparatus of Example 6.

The suspension to be filtered is kept mixed in a second reservoir 12 or process liquid tank (process zone). A first reservoir 14 or buffer vessel (buffer zone), typically a fraction of the volume of the second reservoir 12, is initially filled with solvent (i.e. de-ionised water for aqueous-based filtrations). The first reservoir 14 is substantially airtight such that, when permeate liquid is removed via a permeate outlet means 16 into the second reservoir 12, liquid is drawn into the first reservoir 14 via a first conduit 18 from the second reservoir 12, the latter being a vessel at atmospheric pressure at all times.

During the filtration process, permeate or material passing through a filtration means 50 will be transported back to the second reservoir 12 or process fluid tank, whereas the retained solids or retentate is recirculated in a substantially airtight loop 70 comprising a retentate outlet means 20, the first reservoir 14 and a second conduit 22 and is, thereby, concentrated in the first reservoir 14 or buffer vessel. Solids will remain in suspension in the first reservoir liquid volume and thus prevent clogging up of the filtration means 50.

If required, a relief valve 24 may be positioned on the top cover 26 of the first reservoir 14, which valve 24 can be opened at the end of the process, to permit recovery of dissolved analyte in the buffer liquid. Opening the valve 24 will stop the process liquid flow along the first conduit 18 to the first reservoir 14 and concentration of the buffer liquid will then ensue.

A valve 28 may also be located on the retentate outlet means 20. This valve 28 can be opened to empty the first reservoir 14 after a purification run. Fresh solvent (i.e. de-ionised water) can be fed through a rinse port 30 provided in the first reservoir 14 to clean the apparatus 10 of the present invention.

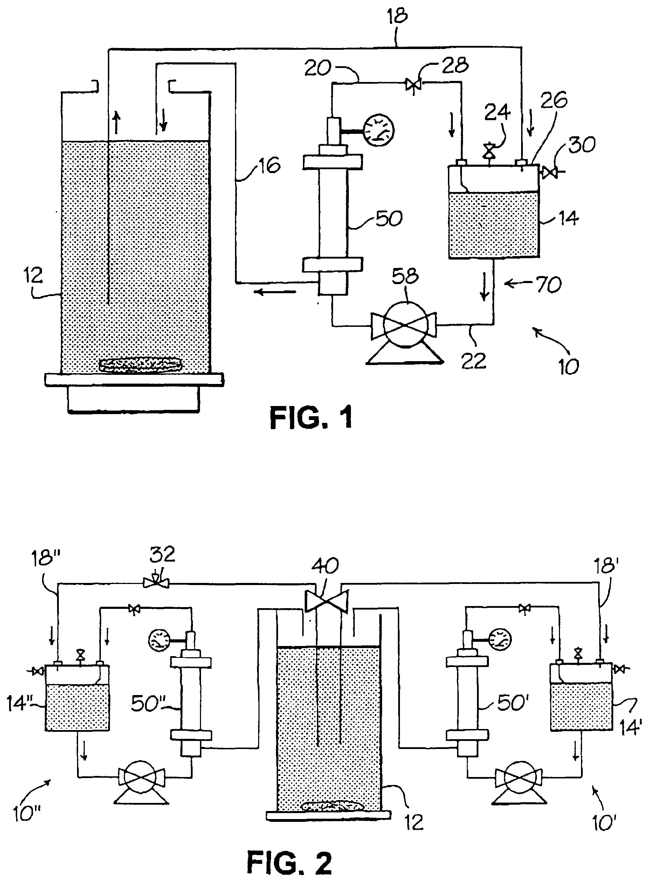

The apparatus 10 as described hereinabove and as illustrated in Figure 1 acts as large scale filtration, as an automated laboratory pre-purification step. The Figure 1 embodiment can be used to batch-wise eliminate particulate matter from turbid solutions. Specifically, particulate matter is concentrated in the first reservoir 14 and clear process liquid is to be found in the second reservoir 12. The filtration process does not require operator intervention as the system does not require, for example, switching off after a clear process liquid is obtained, in contrast to the conventional filtration approaches. By simple replacement of the microfiltration membrane by, for example, an ultrafiltration separator or reverse osmosis filtration means, liquid separations at the molecular scale range can be accomplished as well.

Referring now to Figure 2 of the accompanying drawings, two stand-alone apparatuses 10', 10'' can be inter-connected to allow multiple stage separations, specifically, a microfiltration unit 10' can be connected to an identical unit 10'' fitted with ultrafiltration separation capability. Thus the process fluid is cleaned by microfiltration prior to treatment by ultrafiltration. Analytes of interest for treatment that remain in the first reservoir 14' of the microfiltration unit 10', can be completely recovered and transported to the ultrafiltration device 10'' by simply allowing continuous recirculation of the process fluid in the microfiltration module 10'. Fitting a by-pass valve 32 in the first conduit 18'' (from the second reservoir 12'' to the first reservoir 14'') makes the ultrafiltration module 10'' compatible with a conventional diafiltration mode of operation. A valve (not shown) may be fitted to the permeate outlet means 16', so as to induce a back pressure in the lower half of the filtration means 50'. A valve 40 is fitted to interconnect the respective first conduits 18', 18'' and, thereby, to permit the microfiltration unit 10' and the ultrafiltration unit 10'' to be operated in that sequence. Alternatively, an operator may actuate, firstly, the microfiltration unit 10' and then the ultrafiltration unit 10''.

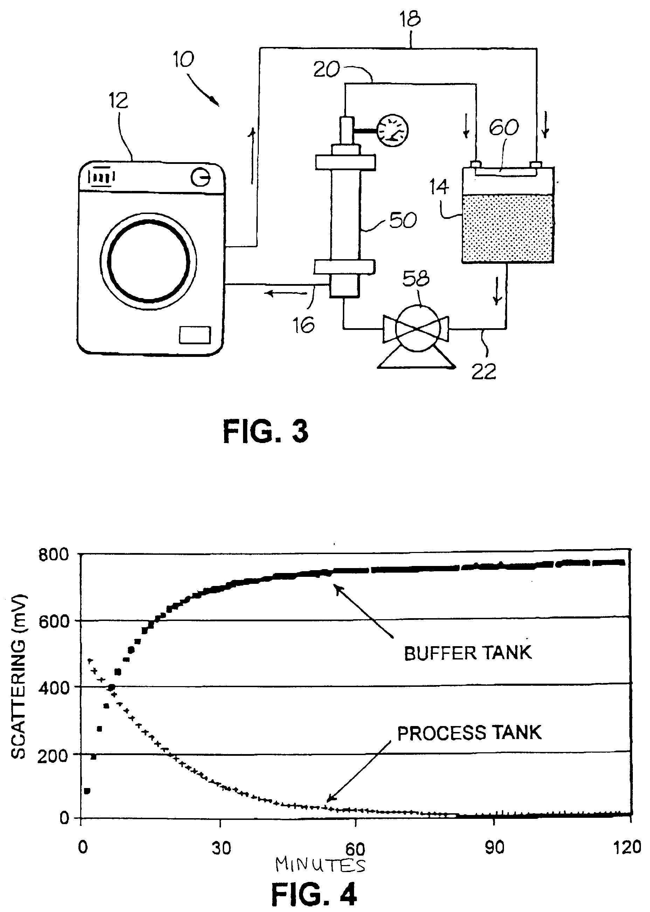

Referring now to Figure 3 of the accompanying drawings, there is illustrated in-line particulates elimination during a wash/cleaning cycle in laundry and dishwashing machines. The fluid conduits of the laundry machine itself comprise the second reservoir 12 and the first conduit 18 feeds wash liquor from the laundry machine 12 to the first reservoir 14. A coarse filter 60 is provided in the first reservoir 14, to prevent clogging with fabric debris or the like. The second conduit 22 feeds fluid from the first reservoir 14 to the filtration means 50, using a pump means or pump 58. Permeate is returned to the laundry machine (or second reservoir 12) via the permeate outlet means 16, whilst retentate is recirculated via the retentate outlet means 20 to the first reservoir 14.

It will be appreciated that the output of the pump 58 will be suitably rated for the type of cross-flow filtration involved.

The Figure 3 embodiment permits efficient particulates removal from the wash liquor and returns substantially undiluted, substantially particulates-free or clean wash liquor to the laundry machine.

The Figure 3 embodiment also permits in-line particulates elimination from aquarium water and, in that event, the aquarium itself would constitute the second reservoir 12.

It will be appreciated that the cross-flow filtration apparatus of the present invention can be fitted with the following options to permit:
- Controlled release of reactants or other compounds into the process zone or time-controlled elimination of reactants or compounds from the process zone;
- Heating/cooling of process liquid;
- Complexing of process liquid compounds (e.g. reactant in buffer vessel liquid or immobilised on membranes);
- Enzymatic reactions - the enzyme being either retained in solution/emulsion in buffer vessel or immobilised on membrane surfaces;
- UV irradiation - UV light can be used to degrade organic compounds. However, application in, for example, a laundry machine could potentially result in fabric damage. This could be eliminated by positioning the UV source in a remote spot such as the buffer zone (first reservoir).

The following benefits are associated with the cross-flow filtration apparatus of the present invention.
**Automation:** Filtration occurs automatically. Retained compounds are transferred to the buffer zone (first reservoir 14) and permeate is transferred to the process zone (second reservoir 12), whilst the process liquid volume remains substantially constant.
**Liquid Transfer:** Processed liquid is returned to the process zone (second reservoir 12). There are no special requirements for this container (an open vessel such as a beaker is suitable) for, for example, analytical applications. The need to recover processed liquid from a special process liquid container is eliminated.
**Recovery of Particulate Matter:** If required, particulate matter can be recovered easily from the buffer zone (first reservoir 14), after the filtration process, in a concentrated form.
**Easy Cleaning and Maintenance:** All conventional cleaning and filter medium restoration procedures can be applied (for example, back flushing, permeate side pressurising, acid/caustic wash, etc.).
**Hyphenation:** The interconnection of two modules operating in different modes (as described in relation to Figure 2 hereinabove for a combined apparatus with micro- and ultrafiltration systems), allows for automated separation of process liquids with increased selectivity.

The present invention will now be exemplified by reference to the following non-limiting examples.

### Example 1

Initial tests with a prototype cross-flow filtration apparatus as illustrated in Figure 1 of the accompanying drawings were carried out to demonstrate the performance of the apparatus of the present invention. The first and second reservoirs had respective volumes of 3000ml and 1000ml. The filtration means had a pore size of 0.1µm and a surface area of 0.03m². The filtration means was supplied by Amicon. As a test process liquid, a 5% emulsion of zeolite in water (pH 10.5) was used, so as to mimic the characteristics of a detergent solution. 2 litres of the emulsion could be processed in one hour - no operator intervention was required during the purification process.

Better than 96% solids (zeolite) removal from the process liquid was obtained. The recovery was measured at various time points by centrifuging aliquots of process and buffer liquid and by weighing the dried pellet material. The performance was also assessed by measuring scattering (turbidity in mV) with process time (in minutes), as illustrated in Figure 4 of the accompanying drawings. It will be observed that solids separation is substantially complete within 60 minutes.

### Example 2

The present cross-flow filtration apparatus in the form of a reversed diafiltration system and operating with a hollow fibre type filtration means of 0.1 µm pore size; a 1.51 second reservoir; and a 0.51 first reservoir, was used to demonstrate its applicability for laundry detergent (liquid and granular detergent) analytical purification.

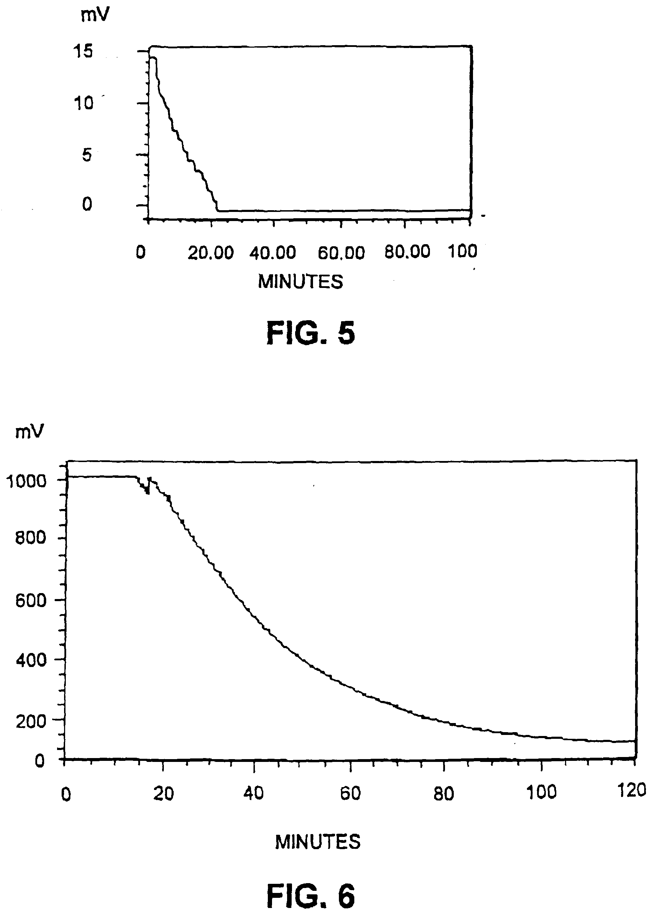

A liquid detergent formulation (Ariel Futur (Trade Mark), prod. Code 247 A41 08:07, in a concentration of 6.67ml/l water)and a zeolite-containing granular detergent formulation (Ariel Color (Trade Mark), prod. Code 417 BA1, in a concentration of 7 g/l water) were each prepared in deionised water. The progress of the filtration was monitored via a phototrode probe (wavelength 650nm) in the process volume (see Figures 5-6 of the accompanying drawings).

Figure 5 shows the filtration progress (absorbance; mV) with time for Ariel Futur (Trade Mark) in deionised water. Substantially complete solids removal is observed after just over 20 minutes.

Figure 6 shows the filtration progress (absorbance; mV) with time for Ariel Color (Trade Mark) in deionised water. The absorbance is off-scale for the first 20 minutes or so, following which a steady filtration progression is observed.

Filtration means' permeation flow rates were restored after each experiment by rinsing with acidified deionised water (10ml HCl/l water)

Figures 5 and 6 are examples of reversed diafiltration data, obtained for both detergent matrices in deionised water. Purification takes longer for the granular detergent, most probably due to the large zeolite load (see Figure 6 in comparison to Figure 5).

No further steps for an analytical multi-stage purification are needed, since the speed of filtration is sufficient to allow a combination of 0.1µm microfiltration with, for example, a 1,000 molecular weight cut-off polymer isolation by ultrafiltration using the Figure 2 inter-connected apparatuses 10', 10''.

### Example 3

In order to demonstrate applicability to laundry wash liquor cleanup, filtration progress was assessed using a pore sized filtration means (0.65µm) of the "hollow fibre" type (surface area 0.013m²; supplied by AG Technology) to treat solutions and suspensions of detergent formulations, with a process zone volume of 0.51 and a buffer zone volume of 0.51.

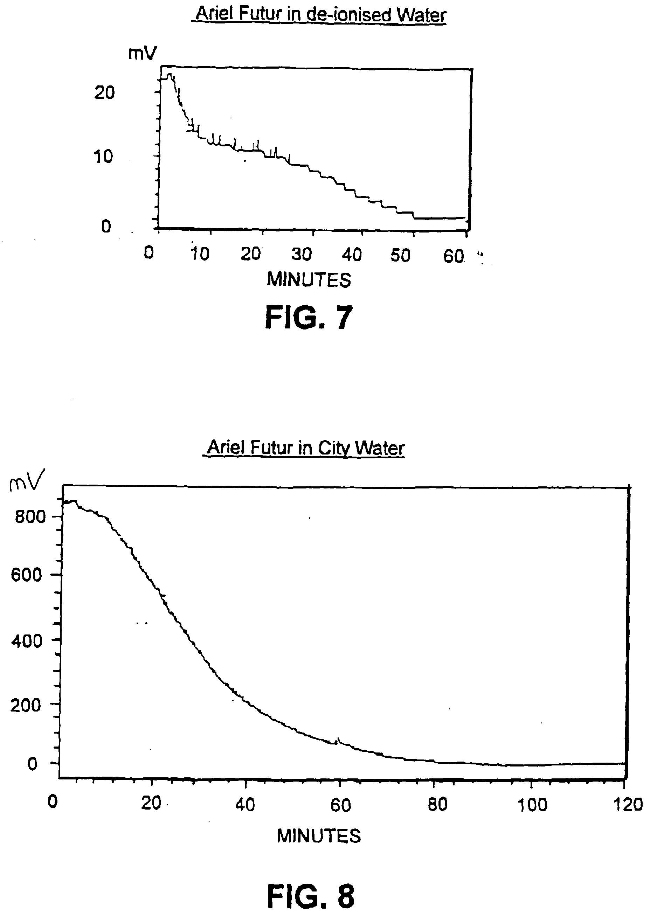

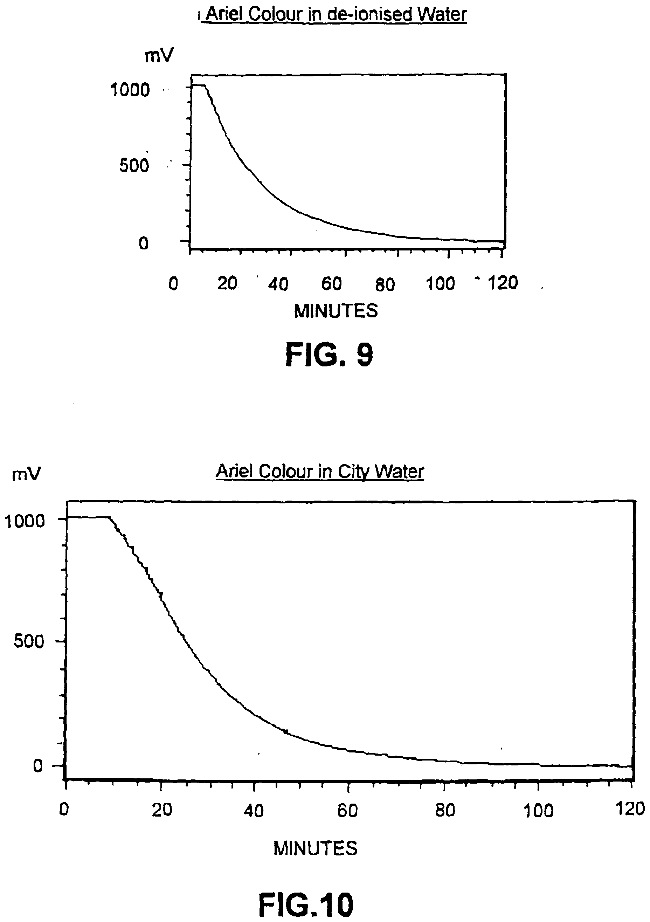

A liquid detergent (Ariel Futur (Trade Mark), prod. Code 247 A41 08:07, in a concentration of 6.5ml/l water) and a granular detergent (Ariel Color (Trade Mark), prod. Code 417 BA1, in a concentration of 7 g/l water) were each prepared, both in deionised water and in city water. Progress of the filtration was monitored via a phototrode probe (wavelength 650nm; absorbance; mV) in the process volume (see Figures 7-10 of the accompanying drawings) Filtration means' permeation flow rates were restored after each experiment by rinsing with acidified deionised water (10 ml HCl/l water).

Compared with Example 2, permeate flow rates are reduced for both deionised and city water based detergent solutions. Even so, filtration is almost complete after 60 to 120 minutes for all conditions tested, although the filtration means' membrane area, at 0.013 m², was much smaller as compared to Example 2 data using a 0.1µm filtration means (0.03m², Amicon H1MP01-43).

For laundry washing machine applications, surface areas of between 0.25 and 0.5 m² are normally required, assuming equal volume ratios between process and buffer vessels, as in the analytical scale experiments conducted in this example.

For practical reasons, a minimum first reservoir or buffer vessel volume of 500ml is normally required to allow sufficiently high cross-flow rates across the filtration means and to ensure thorough mixing of process fluid and buffer vessel contents.

As can be seen from the phototrode traces (Figures 7-10), extremely high filtration efficiencies can be achieved (deionised water was used to set the blank value at zero response). HDL type detergent (Ariel Futur (Trade Mark) in city water) can be processed in about half an hour, whereas a zeolite-containing granular detergent (Ariel Color(Trade Mark) in city water) takes approximately one hour to complete.

Filtration was progressed at back pressures of 0.2 bar or lower. A low operating pressure is important as it enables the cross-flow filtration apparatus of the present invention to be run with a low-cost centrifugal pump (e.g. of the type used in laundry washing machines).

Liquid volumes used in laundry machines are usually about 15-20 litres (typically 161). In order to complete particulate removal in typical laundry washing machine process times (50-90 minutes, depending on machine water feed temperature and wash program), the required filtration means' surface area is preferably at least 0.25m².

### Example 4

The present example concerns filtration, using a laundry wash liquor as the process fluid.

The liquid detergent (Dash Color Liquor (Trade Mark) was assessed using, as the process fluid, the output from a 60°C wash in a Zanussi-JetSystem (Trade Mark) laundry washing machine operated under typical in-home conditions following the manufacturer's recommendations as to laundry load and product usage.

The filtration means was of the "hollow fibre" type (having a pore size of 0.65µm; a surface area of 0.013m² and supplied by AG Technology. The filtration equipment was identical to that in Example 3.

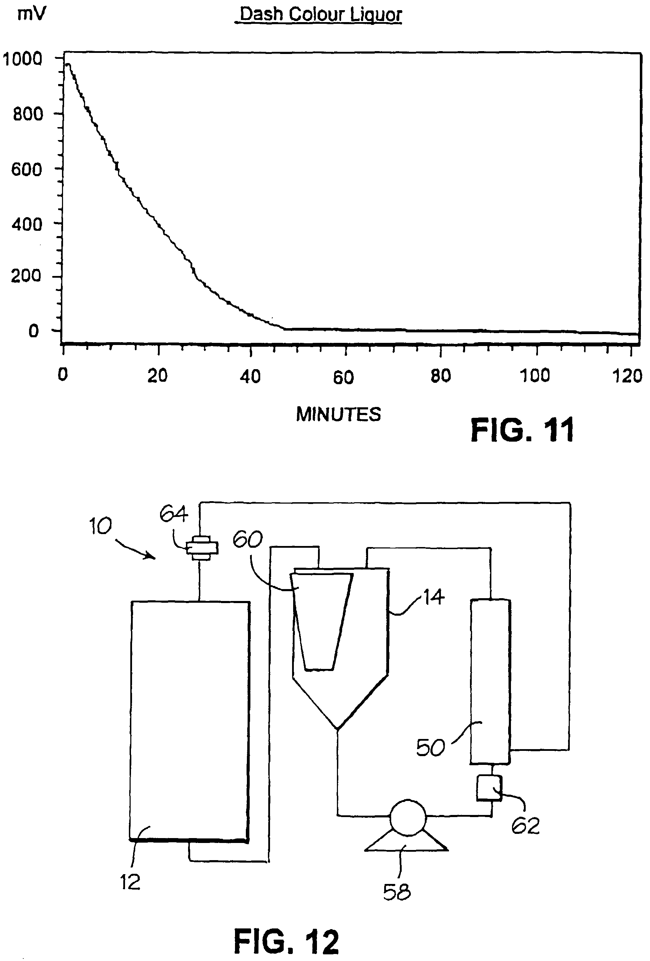

Figure 11 of the accompanying drawings shows the filtration progression with time, by plotting the absorbance (mV) of the wash liquor (as measured by a 650nm phototrode) versus filtration time.

It will be observed that filtration separation is virtually complete at just over 45 minutes.

Thus, successful particulate removal has been carried out within a typical laundry washing machine process time of 50-90 minutes, more specifically, in just under 50 minutes.

The process can also be undertaken with larger pore sized (i.e. 1-1.5µm) filtration means for additional purification speed and with filtration means in the form of plate and frame devices, rather than hollow fibre modules which are limited with respect to pore size.

### Example 5

Figure 12 of the accompanying drawings illustrates an optimised cross-flow filtration apparatus of the present invention.

It will be appreciated that filtration speed and/or filtration means' load of the apparatus of Example 4 is further improved by incorporating a disposable pre-filter device 60 in the first reservoir 14 to retain part of the particulate load in the first reservoir 14 or buffer vessel. Such a pre-filter device 60 also serves to prevent entry of larger sized debris (e.g. fabric fragments) into the narrow channels of cross-flow filtration apparatus 10 itself. Additionally or alternatively, an in-line, pre-filter device 62 is located intermediate the re-circulation pump 58 and the filtration means 50, allowing the pump's pressure to force liquid through the in-line pre-filter device 62.

### Example 6

Example 6 concerns filtration progression, by manually applying an intermittent filtration membrane clean-sweeping step.

Filtration means' efficacy and life-span is increased by applying an intermittent filtration membrane clean-sweeping step. This involves incorporating a low cost pulsating "pinch valve" 64 in the permeate outlet means 16, which valve 64 is intermittently closed during the filtration process. The result is an increased back pressure at the lower half of the filtration membrane's permeate side. This causes retained matter to be released from the membrane at the pressure side, which retained matter is subsequently swept away by the cross-flow liquid in the filtration means 50. Simulating this effect by manually shutting off the permeate outlet means 16 is an effective way to improve the overall performance of the cross-flow apparatus of the present invention 10.

As in Example 4, the detergent formulation is Dash Color Liquor (Trade Mark) in a 60°C wash liquor from a Zanussi-JetSystem (Trade Mark) laundry washing machine.

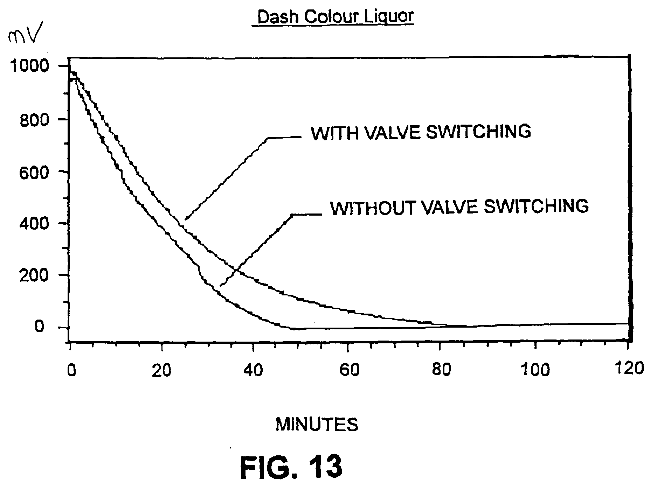

Figure 13 shows the filtration progression (absorbance; mV versus time) with and without a manual switching regime. The manual switching regime chosen was a frequency of 0.03 minutes and an open-closed ratio of 1:1.

It should be noted that, although overall filtration progression is faster without valve switching, the speed of filtration is enhanced during the filtration phase of the manual switching regime.

### Example 7

The present example demonstrates surfactant separation from Ariel Futur (Trade Mark) and Ariel Color (Trade Mark) detergent formulations in both city water and deionised (Milli-Q (Trade Mark)) water. The filtration means is a hydrophobic polysulfone membrane.

| **SURFACTANT REMOVAL (meq/mL)** | | | | | |
|---|---|---|---|---|---|
| **Product Type** | **Filter Type (µm)** | **City Water** | | **Milli-Q Water** | |
| | | **Buffer** | **Process** | **Buffer** | **Process** |
| Ariel Futur | 0.1 | ₋ | ₋ | 0.05936 | 0.01375 |
| Ariel Color | 0.1 | ₋ | ₋ | 0.03679 | 0.003875 |
| Ariel Futur | 0.65 | 0.04506 | 0.02103 | 0.01865 | 0.01794 |
| Ariel Color | 0.65 | 0.02254 | 0.01006 | 0.02910 | 0.003488 |

## Claims

1. A cross-flow filtration apparatus (10) suitable for connection to a process zone (12) for filtration of process fluid, the apparatus comprising a cross-flow filtration means (50) having a feed inlet means (18, 22), a permeate outlet means (16) and a retentate outlet means (20) in fluid communication therewith, characterised in that the apparatus (10) comprises a buffer zone (14); in that the feed inlet means (18, 22) includes first and second conduits, the first conduit (18) being in fluid communication from the process zone (12) to the buffer zone (14), and the second conduit (22) being in fluid communication from the buffer zone (14) to the filtration means (50), in that the retentate outlet means (20) is in fluid communication with the buffer zone (14); and in that the permeate outlet means (16) is in fluid communication with the process zone (12).

2. A cross-flow filtration apparatus (10) according to Claim 1, characterised in that the buffer zone (14) is adapted and arranged such that, at steady state, outflow along the permeate outlet means (16) is substantially equivalent (mass-balanced) to inflow along the first conduit (18).

3. A cross-flow filtration apparatus (10) according to Claim 2, in which the buffer zone (14) is substantially closed to atmosphere.

4. A cross-flow filtration apparatus (10) according to Claim 3, in which the retentate outlet means (20), the buffer zone (14) and the second conduit (22) form, with the filtration means (50), a closed retentate recirculating loop (70).

5. A cross-flow filtration apparatus (10) according to any one of the preceding claims, in which the filtration means (50) is adapted for filtration of liquids and is, preferably, selected from a microfiltration, ultrafiltration or reverse osmosis filtration means (50).

6. A cross-flow filtration apparatus (10) according to any one of Claims 1-4, in which the filtration means (50) is adapted for filtration of gases.

7. A cross-flow filtration apparatus (10) according to any one of the preceding claims, in which two or more first conduits (18), buffer zones (14), second conduits (22), filtration means (50), retentate outlet means (20) and permeate outlet means (16) are in fluid communication with one process zone (12).

8. A cross-flow filtration apparatus (10) according to Claim 7, in which a valve (40) is provided in operative association with the two or more first conduits (18).

9. A cross-flow filtration apparatus (10) according to any one of Claims 1-5, in which the process zone (12) comprises liquid conduits of a laundry or dishwashing machine or an aquarium.

10. A domestic appliance, preferably a laundry or dishwashing machine incorporating a cross-flow filtration apparatus (10) according to any one of the preceding claims.

11. A chemical reactor, enzyme reactor or bioreactor incorporating a cross-flow filtration apparatus according to any one of Claims 1 to 8.

12. Electrocoating or electroplating equipment incorporating a cross-flow filtration apparatus according to any one of Claims 1 to 8.

13. Blood/plasma fractionation or filtration equipment incorporating a cross-flow filtration apparatus according to any one of Claims 1 to 8.

14. Air/water/effluent treatment equipment incorporating a cross-flow filtration apparatus according to any one of Claims 1 to 8.

15. Use of a cross-flow filtration apparatus (10) suitable for connection to a process zone (12) for the filtration of process fluid, the apparatus comprising a buffer zone (14), a cross-flow filtration means (50) having a feed inlet means (18, 22), a permeate outlet means (16) and a retentate outlet means (20) in fluid communication therewith, and wherein the feed inlet means (18, 22) includes first and second conduits, characterised in that, in use, the first conduit (18) is in fluid communication from the process zone (12) to the buffer zone (14); the second conduit (22) is in fluid communication from the buffer zone (14) to the filtration means (50); the retentate outlet means (20) is in fluid communication with the buffer zone (14); and the permeate outlet means (16) is in fluid communication with the process zone (12).
